Europäisches Patentamt

**European Patent Office**

**Office européen des brevets**

(19)

(11) Publication number: **0 190 937**
**A2**

(12) # EUROPEAN PATENT APPLICATION

(21) Application number: **86300829.8**

(22) Date of filing: **06.02.86**

(51) Int. Cl.⁴: **C 07 C 102/04**
**C 07 C 102/06**

(30) Priority: **08.02.85 GB 8503279**
**28.06.85 GB 8516433**

(43) Date of publication of application:
**13.08.86 Bulletin 86/33**

(84) Designated Contracting States:
**BE DE FR GB IT NL SE**

(71) Applicant: **BP Chemicals Limited**
**Belgrave House 76 Buckingham Palace Road**
**London, SW1W 0SU(GB)**

(72) Inventor: **Gulliver, David Jeffrey**
**BP Chemicals Limited Saltend**
**Hedon Hull, HU12 8DS(GB)**

(74) Representative: **Fawcett, Richard Fennelly et al,**
**BP INTERNATIONAL LIMITED Patents Division Chertsey**
**Road**
**Sunbury-on-Thames Middlesex, TW16 7LN(GB)**

(54) Carbonylation processes using amines or ammonia.

(57) a process for the preparation of amides or nitriles is provided. The process comprises carbonylating a nitrogen-containing compound of formula $R^1R^2NH$, $C_1$ to $C_{20}$ alkyl groups and $C_1$ to $C_{20}$ aryl groups, using a Group VIII noble metal catalyst, a halide-containing promoter and an acid. Examples of the Group VIII noble metals which can be used as catalysts are rhodium and iridium.

EP 0 190 937 A2

1

## CARBONYLATION PROCESSES USING AMINES OR AMMONIA

The present invention relates to a process for preparing nitrogen-containing compounds. More specifically, this invention relates to a process for preparing nitrogen-containing compounds by the carbonylation of ethers and/or esters and reactive nitrogen containing compounds in the presence of a catalyst system comprising a Group VIII noble metal component, a halide containing compound and an acid.

Carbonylation reactions whether with or without the presence of catalytic components are well known, see for example 'Carbon Monoxide in Organic Synthesis' by J. Falbe. However, the carbonylation of mixtures of (1) esters and/or ethers and (2) nitrogen-containing compounds has not been previously reported.

Accordingly, the present invention provides a process for the preparation of amides, nitriles or mixtures thereof which process comprises reacting (1) an ether, ester or mixture thereof with (2) a nitrogen-containing compound having the formula $R^1 R^2 NH$, wherein $R^1$ and $R^2$ are selected from hydrogen, $C_1$ to $C_{20}$ alkyl groups and $C_1$ to $C_{20}$ acyl groups, and (3) carbon monoxide at elevated temperature in the presence of a catalyst comprising a Group VIII noble metal, a halide-containing promoter and an acid.

As regards the amide products these can be mono-, di- or triamides. Preferably the amide is selected from acetamide, N-methyl acetamide, N-ethyl acetamide, N-propyl acetamide, diacetamide, N-methyl diacetamide, N-acetyl acetamide and N-acetyl-N-methyl acetamide. The nitriles are preferably $C_1$, to $C_4$

0190937

nitriles.

The ethers used as first reactant in the present process are suitably those having the formula ROR wherein the R groups are independently $C_1$ to $C_{20}$ alkyl groups. Of these ethers, those having $C_1$ to $C_4$ alkyl groups such as dimethyl ether, diethyl ether, methyl ethyl ether and methyl propyl ether are preferred.

When an ester is used as a first reactant either together with or as an alternative to, an ether, it is one suitably having the formula RCOOR wherein the R groups are independently $C_1$ to $C_{20}$ alkyl groups preferably $C_1$ to $C_4$ alkyl groups. Preferred esters include methyl acetate, ethyl acetate, methyl propionate, ethyl propionate and the like.

The second reactant is a nitrogen-containing compound of formula $R^1 R^2 NH$ wherein $R^1$ and $R^2$ are selected from hydrogen, $C_1$ to $C_{20}$ alkyl groups and $C_1$ to $C_{20}$ acyl groups. When $R^1$ or $R^2$ are alkyl groups, they are preferably $C_1$ to $C_4$ alkyl groups and when they are acyl groups they are preferably $C_1$ to $C_4$ acyl groups. The second reactant is most preferably selected from ammonia, methylamine, ethylamine, 2-butylamine, dimethylamide, acetamide, propionamide and the like.

The second reactant can also be generated in situ under the reaction conditions by use of a precursor instead of the second reactant. Such precursors include quaternary salts of the second reactant for example acid addition salts of formula $R^1 R^2 NH_2^+ X^-$ wherein $X^-$ is an anion derived from an acid by removal of a proton. Example of $X^-$ include chloride, bromide or iodide ion, acetate ion formate ion and the like. It will be appreciated that by using a quaternary iodide salt it is possible to add both the precursor of the second reactant and the iodide promoter at the same time.

The carbonylation reaction occurs by contacting the appropriate mixture of ether, ester, and nitrogen-containing compound with carbon monoxide. While the carbon monoxide reactant can contain some $CO_2$, hydrogen or other impurities, the carbon monoxide reactant is preferably one containing high concentrations of carbon monoxide. Most preferred are carbon monoxide sources which contain greater than 90 mole % carbon monoxide.

The process of this invention occurs in the presence of a catalyst system comprising a Group VIII noble metal component, and a halide-containing promoter. The Group VIII noble metal component is selected from the metals ruthenium, rhodium, palladium, osmium, iridium, platinum or combinations thereof with rhodium or iridium being preferred. The noble metal component can be conveniently added to the reaction mixture as a metal, a salt, or a complex such as a carbonyl complex. Preferably, the Group VIII metal is added as a metal salt such as as a metal acetate, nitrate, or halide.

While the noble metal component is suitably used in a form soluble in the reaction mixture, insoluble or supported noble metal components can also be employed. For example, the noble metal component, suitably a metal or a metal complex, can be supported on any of the well known inert supports such as silica, alumina, silica/alumina, clay,zirconia, carbides, graphites, zeolites, polymers and the like. Any of the conventional techniques for preparing the heterogeneous noble metal component such as impregnation, precipitation or ion exchange techniques which are well known to those skilled in the art can be employed to prepare such supported material.

The halide-containing promoter is suitably either a bromide - or iodide-containing compound. Such compounds include bromine $Br_2$, iodine $I_2$, hydrogen bromide, hydrogen iodide, organic bromides, organic iodides, metal bromides and metal iodides and quaternary ammonium or alkylammonium bromides or iodides. Conveniently such bromide - or iodide-containing compounds are $C_1$ to $C_4$ alkyl bromides or iodides of which methyl iodide is preferred. The mole ratio of the Group VIII noble metal to halide-containing promoter is suitably in the range 1:10 to 1:1000 preferably 1:50 to 1:200.

The reaction can optionally be carried out in the presence of an acid. The acid can be any of the well known inorganic or organic acids such as hydrochloric, hydrobromic, nitric, sulphuric, acetic, carbonic, formic, acetic, oxalic and the like. Preferably, the acid is an organic acid such as acetic or formic acid. The acid component is generally added in amounts up to 30% by weight of the reactants.

Although the process of the present invention can be carried out in the gas phase, it is preferably carried out in the liquid phase. However, if ammonia or a volatile amine is used, it can be supplied to the reaction mixture in the gaseous phase either separate from or in conjunction with the carbon monoxide reactant.

As regards reaction conditions, the reaction is preferably carried out at a temperature in the range 150°C to 200°C, most preferably 170°C-200°C. The reaction is also suitably carried out at a superatmospheric pressure in the range 4 to 200 bars. The superatmospheric pressure can be created, for example, by using an overpressure of the gaseous reactants such as carbon monoxide. The reaction can be carried out either batchwise or continuously

The present invention is further illustrated by the following Examples. However, it is to be understood that the scope of this invention includes equivalent embodiments, variations and modifications.

Example 1

Acetic acid (20.3g), methyl iodide (23.5g) and rhodium acetate (0.34g) were charged to a Hastelloy autoclave which was pressurised with ammonia (70 psig). Dimethyl ether (12.5 ml) was distilled into the previously cooled autoclave and the autoclave allowed to warm up to room temperature. The autoclave was pressurised with carbon monoxide (1500 psig). The contents were stirred for 2 hours at 185°C. At this temperature the initial total pressure was approximately 1800 psig. Gas chromatography (GC) analysis of the reactor contents, after cooling to room temperature, showed 11% by weight diacetamide. Minor products (less than 3% by weight each) include N-methyl diacetamide, N,N-dimethylacetamide, N-methylacetamide, acetamide and acetonitrile.

Example 2

Example 1 was repeated with a higher dimethyl ether and ammonia concentration at a lower pressure and catalyst concentration. Dimethyl ether (25 ml), acetic acid (10.2g), methyl iodide (12.0g), and rhodium acetate (0.15g) were charged to the autoclave which was then pressurised with ammonia (60 psig) and carbon monoxide (900 psig). At the end of the experiment, GC analysis showed the reaction mixture contained 10% by weight of diacetamide.

Example 3

Acetic acid (20.6g), methyl iodide (23.3g), rhodium acetate (0.30g) and methylamine (8 ml) were charged to a Hastelloy autoclave. The autoclave was cooled and dimethyl ether (12.5 ml) distilled into the autoclave which was then allowed to warm up to room temperature. The autoclave was pressurised with carbon monoxide (1100 psig). The contents were then stirred for 2 hours at 185°C. GC analysis of the reactor contents, after cooling to room temperature, showed the presence of N-methyldiacetamide 8.2% by weight.

Example 4

Example 3 was repeated except with a higher dimethyl ether concentration and a reduced catalyst concentration. Dimethyl ether (20 ml), acetic acid (20.6g), methyl iodide (24.0g), methylamine (8 ml) and rhodium acetate (0.30g) were charged to the autoclave which was then pressurised with carbon monoxide (1200 psig). At the end of the experiment, GC analysis showed the reaction mixture contained 11% by weight of N-methyldiacetamide.

Example 5

Acetic acid (2.0g), ethyl iodide (12.3g), rhodium acetate (0.15g), ammonium acetate (9.0g) were charged to a Hastelloy autoclave. The autoclave was cooled and dimethyl ether (25 ml) was distilled into the autoclave which was then allowed to warm up to room temperature. The autoclave was pressurised with carbon monoxide (900 psig). The contents were then stirred for 2 hours at 185°C. GC analysis of the reactor contents, after cooling to room temperature, showed the presence of diacetamide 6% by weight.

Example 6

Methyl acetate (28 g), methyl iodide (12.5 g), acetic acid (8 g) and rhodium acetate (0.15 g) were charged to a Hastelloy autoclave which was then pressurised with ammonia (80 psig) and carbon monoxide (1100 psig). The contents were then stirred for 2 hours at 185°C. At this temperature, the initial total pressure was approximately 1300 psig. Gas Chromatography (GC) analysis of the reactor contents, after cooling to room temperature, showed 17% by weight diacetamide. Minor products (less than 8% weight each) include N-methyl diacetamide, N,N-dimethylacetamide N-methyl acetamide and acetonitile. (These minor products were also identified in Examples 2 to 5).

Example 7

Example 6 was repeated except with a carbon monoxide pressure of 400 psig and a low partial pressure of hydrogen (75 psig) was used. At the end of the experiment, GC analysis showed 17% by weight diacetamide.

Example 8

Example 6 was essentially repeated except that $RhCl_3.3H_2O$ (0.18 g) was substituted for the rhodium acetate. GC analysis of the product showed 7% by weight diacetamide.

Example 9

Methyl acetate (27 g), methyl iodide (11 g), acetic acid (1.4 g), ammonium acetate (9 g) and rhodium acetate (0.15 g) were charged to a Hastelloy autocalve which was then pressurised with carbon monoxide (400 psig) and hydrogen (75 psig). The contents were then stirred for 2 hours at 185°C. GC analysis showed 15% by weight diacetamide.

Example 10

Methyl acetate (19 g), methyl iodide (11 g), acetic acid (4 g), acetamide (12 g) and rhodium acetate (0.15 g) were charged to a Hastelloy autoclave which was then pressurised with carbon monoxide (860 psig) and hydrogen (75 psig). The contents were then stirred for 4 hours at 185°C. Diacetamide 20% by weight was identified at the end of the reaction by GC analysis.

Example 11

Methyl acetate (30g), methyl iodide (13g) and rhodium acetate (0.15g) were charged to a Hastelloy autoclave which was then pressurised with ammonia (80 psig) and carbon monoxide (1000 psig).The contents were then stirred for 2 hours at 200°C. At the end of the experiment, GC analysis showed 11% by weight diacetamide.

Example 12

Example 6 was repeated except that tetramethylammonium iodide [Me₄N]I (8 g) was substituted for methyl iodide. GC analysis of the product showed 12% by weight diacetamide.

Example 13

Example 6 was repeated except that methylamine was (6 g) substituted for ammonia. At the end of the experiment, GC analysis showed 15% by weight N-methyl diacetamide.

**Claims:**

1. A process for the preparation of amides, nitriles or mixtures thereof which process comprises reacting (1) an ether, ester or mixture thereof with (2) a nitrogen-containing compound having the formula $R^1R^2NH$, wherein $R^1$ and $R^2$ are selected from hydrogen, $C_1$ to $C_{20}$ alkyl groups and $C_1$ to $C_{20}$ acyl groups, and (3) carbon monoxide at elevated temperature in the presence of a catalyst comprising a Group VIII noble metal, a halide-containing promoter and an acid.

2. A process as claimed in claim 1 wherein the Group VIII noble metal component is either rhodium or iridium.

3. A process as claimed in claim 1 wherein the ester is one having the formula RCOOR wherein the R groups are independently $C_1$ to $C_4$ alkyl groups.

4. A process as claimed in claim 1 wherein the ether is one having a formula ROR wherein the R groups are independently $C_1$ to $C_4$ alkyl groups.

5. A process as claimed in claim 1 wherein the Group VIII noble metal is rhodium and the halide containing promoter is methyl iodide.

6. A process as claimed in claim 1 wherein the amide is diacetamide, the ester is methylacetate and the nitrogen-containing compound is ammonia.

7. A process as claimed in claim 1 wherein the amide is N-methyl diacetamide, the ester is methyl acetate and the nitrogen-containing compound is methylamine.